# EUROPEAN PATENT SPECIFICATION

(11) **EP 2 964 783 B1**
(45) Date of publication and mention of the grant of the patent: **01.08.2018**
(21) Application number: 14714944.7
(22) Date of filing: 06.03.2014
(51) Int. Cl.: C12Q 1/6883, C12N 15/113

(54) **DIAGNOSIS AND TREATMENT OF METABOLIC DISORDERS**
DIAGNOSE UND BEHANDLUNG VON STOFFWECHSELERKRANKUNGEN
DIAGNOSTIC ET TRAITEMENT DES TROUBLES MÉTABOLIQUES

(30) Priority: 06.03.2013 EP 13158015; 06.03.2013 US 201361773210 P
(43) Date of publication of application: 13.01.2016
(73) Proprietor: Royal College of Surgeons in Ireland, Dublin D2 (IE)
(72) Inventor: PREHN, Jochen, Dublin D2 (IE); BYRNE, Maria, Dublin D7 (IE)
(74) Representative: Purdy, Hugh Barry
(86) International application number: PCT/EP2014/054381
(87) International publication number: WO 2014/135653

(56) References cited:
- WO-A2-2009/043353
- L. A. RAMON ET AL: "microRNAs related to angiogenesis are dysregulated in endometrioid endometrial cancer", HUMAN REPRODUCTION, vol. 27, no. 10, 17 August 2012 (2012-08-17), pages 3036-3045, XP055071148, ISSN: 0268-1161, DOI: 10.1093/humrep/des292
- M. SHEPHERD ET AL: "Genetic testing in maturity onset diabetes of the young (MODY): a new challenge for the diabetic clinic", PRACTICAL DIABETES INTERNATIONAL, vol. 18, no. 1, 1 January 2001 (2001-01-01), pages 16-21, XP055071183, ISSN: 1357-8170, DOI: 10.1002/pdi.108
- S. S. FAJANS ET AL: "MODY: History, genetics, pathophysiology, and clinical decision making", DIABETES CARE, vol. 34, no. 8, 25 July 2011 (2011-07-25), pages 1878-1884, XP055071021, ISSN: 0149-5992, DOI: 10.2337/dc11-0035
- SIOBHAN BACON ET AL: "Serum levels of pancreatic stone protein (PSP)/reg1A as an indicator of beta-cell apoptosis suggest an increased apoptosis rate in hepatocyte nuclear factor 1 alpha (HNF1A-MODY) carriers from the third decade of life onward", BMC ENDOCRINE DISORDERS, BIOMED CENTRAL LTD., LONDON, GB, vol. 12, no. 1, 18 July 2012 (2012-07-18), page 13, XP021115731, ISSN: 1472-6823, DOI: 10.1186/1472-6823-12-13
- CLAUDIANE GUAY ET AL: "Diabetes mellitus, a microRNA-related disease?", TRANSLATIONAL RESEARCH, ELSEVIER, AMSTERDAM, NL, vol. 157, no. 4, 18 January 2011 (2011-01-18), pages 253-264, XP028368087, ISSN: 1931-5244, DOI: 10.1016/J.TRSL.2011.01.009 [retrieved on 2011-01-25]
- HERRERA B M ET AL: "Global microRNA expression profiles in insulin target tissues in a spontaneous rat model of type 2 diabetes", DIABETOLOGIA ; CLINICAL AND EXPERIMENTAL DIABETES AND METABOLISM, SPRINGER, BERLIN, DE, vol. 53, no. 6, 3 March 2010 (2010-03-03), pages 1099-1109, XP019797135, ISSN: 1432-0428
- "miR224 is present on Affymetrix miRNA 2.0 array used in XP055071148", , 1 October 2012 (2012-10-01), XP055119429, Retrieved from the Internet: URL:http://www.affymetrix.com/estore/analy sis/index.affx;jsessionid=34C8055D806C59EE 4C7D824E17C551AE.prd11com?category=34005&c ategoryIdClicked=34005&rootCategoryId=3400 5&navMode=34005&aId=netAffxNav [retrieved on 2014-05-22]
- "miR551b is present on Affymetrix miRNA 2.0 array used in XP055071148", , 1 October 2012 (2012-10-01), XP055119430, Retrieved from the Internet: URL:http://www.affymetrix.com/estore/analy sis/index.affx;jsessionid=34C8055D806C59EE 4C7D824E17C551AE.prd11com?category=34005&c ategoryIdClicked=34005&rootCategoryId=3400 5&navMode=34005&aId=netAffxNav [retrieved on 2014-05-22]
- "miR103 is present on Affymetrix miRNA 2.0 array used in XP055071148", , 1 October 2012 (2012-10-01), XP055119435, Retrieved from the Internet: URL:http://www.affymetrix.com/estore/analy sis/index.affx;jsessionid=34C8055D806C59EE 4C7D824E17C551AE.prd11com?category=34005&c ategoryIdClicked=34005&rootCategoryId=3400 5&navMode=34005&aId=netAffxNav [retrieved on 2014-05-22]
- "miR503 is present on Affymetrix miRNA 2.0 array used in XP055071148", , 1 October 2012 (2012-10-01), XP055119647, Retrieved from the Internet: URL:http://www.affymetrix.com/estore/analy sis/index.affx;jsessionid=34C8055D806C59EE 4C7D824E17C551AE.prd11com?category=34005&c ategoryIdClicked=34005&rootCategoryId=3400 5&navMode=34005&aId=netAffxNav [retrieved on 2014-05-22]
- "miR539 is present on Affymetrix miRNA 2.0 array used in XP055071148", , 1 October 2012 (2012-10-01), XP055119649, Retrieved from the Internet: URL:http://www.affymetrix.com/estore/analy sis/index.affx;jsessionid=34C8055D806C59EE 4C7D824E17C551AE.prd11com?category=34005&c ategoryIdClicked=34005&rootCategoryId=3400 5&navMode=34005&aId=netAffxNav [retrieved on 2014-05-22]

## Description

### Background to the Invention

Type 2 diabetes is a problem of enormous and increasing health significance, and no current treatment influences the progression of diabetes. Certain forms of diabetes are associated with alterations in a single gene which makes them ideal systems to study disease pathophysiology, disease progression, therapy responses, and to discover novel biomarkers and novel therapeutics. They are diagnosed very young (<25 years old), run in families, and are often mixed up with type 1 and type 2 diabetes patients. These monogenic forms of diabetes are referred to as Maturity-onset-diabetes-of-the-young (MODY). Individuals with MODY are at significant risk of developing diabetic complications such as heart attacks and strokes, and require specific treatments. They are able to control their diabetes very well with sulfonylurea drugs, but are often treated inappropriately with insulin injections due to their early onset. Genetic testing for this diabetes is expensive and not widely available, and the identification of novel single nucleotide polymorphisms (SNP) in such genes does not automatically indicate whether these alterations have functional consequences. Therefore new and sensitive blood or urine-based tests are required that identify MODY patients.

Many genes mutated in MODY patients alter the expression of specific genes in the affected organs. HNF1A-MODY which accounts for 60 % of all MODY is caused by mutations in the *transcription factor 1 (tcf-1)*/*hepatocyte nuclear factor 1a* (*hnf1a*) gene. It can be characterized as an early-onset form of non-insulin dependent diabetes. HNF1A-MODY accounts for 1-2 % of all diabetes cases however the true prevalence of HNF1A-MODY is not known for most populations, and there is also increased evidence for an important role of *de novo* mutations in HNF1A-MODY patients. Identification of HNF1A-MODY patients allows prompt and optimal treatment (sulphonylurea sensitivity), screening of family members and prevention of diabetic complications. Awareness of the clinical phenotype of HNF1A-MODY and other forms of MODY and availability of molecular testing still remain poor, and vary in different regions. Treatment options and clinical outlook for MODY depends on the exact genetic cause, country of residence, financial cost of molecular testing or referral rates, and may differ greatly from those of type 1 and type 2 diabetes (1). A recent study carried out in UK suggests that approximately 80 % of MODY patients are not diagnosed by genetic testing (1) or misdiagnosed as having type 1 or type 2 diabetes. Consequently, thousands of people with MODY may not receive appropriate treatment for their diabetes, and limited resources for molecular testing may also be putting relatives at high risk for undiagnosed diabetes. Deep sequencing is not readily available in many countries and hospitals and does not provide a functional readout of the SNP identified

It is an object of the invention to overcome at least one of the above-referenced problems.

### Statements of Invention

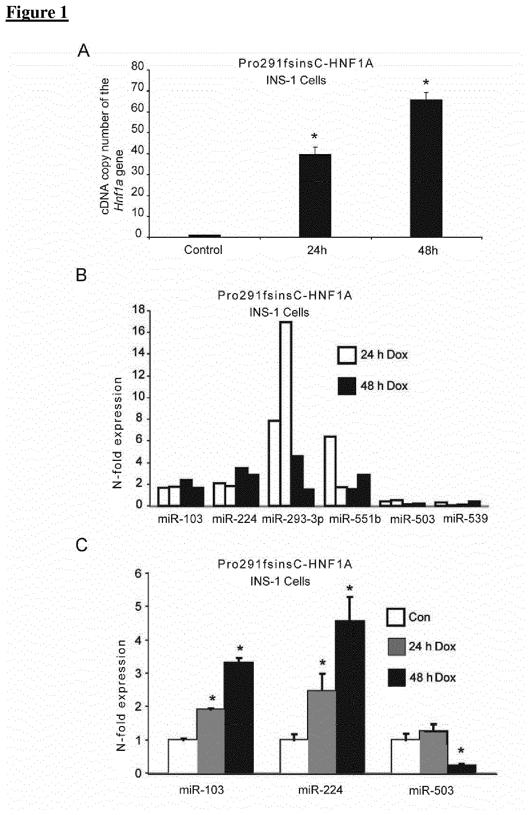

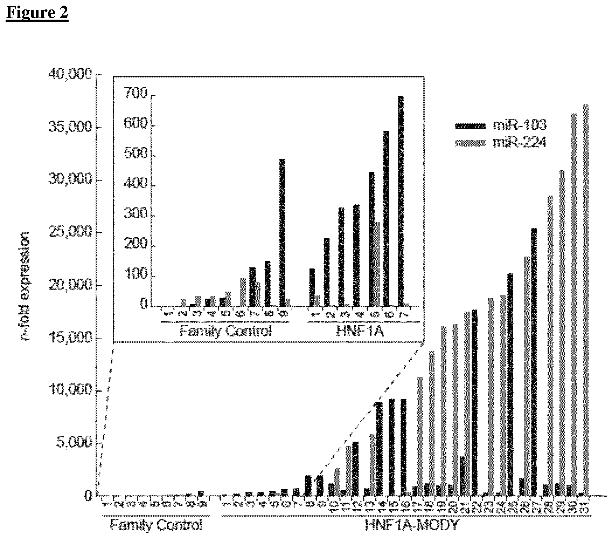

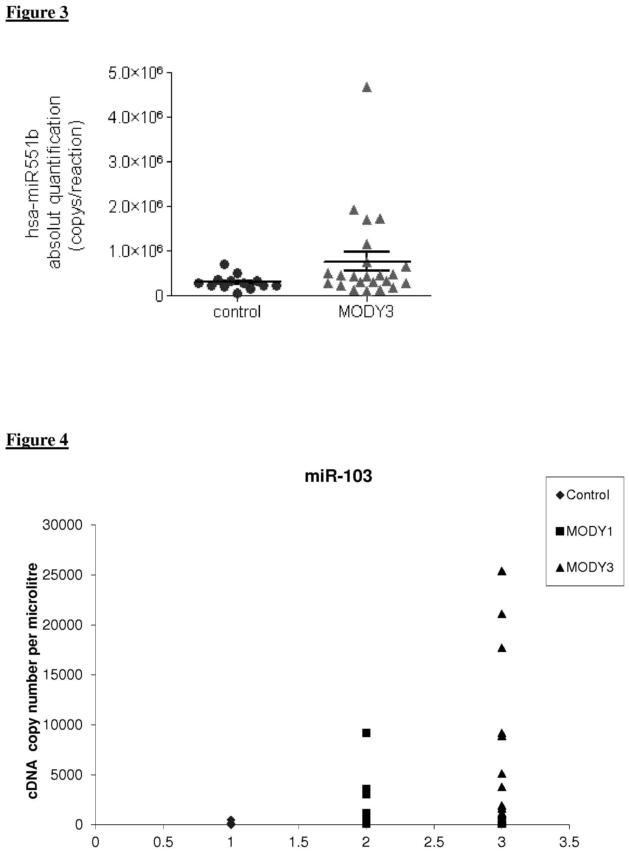

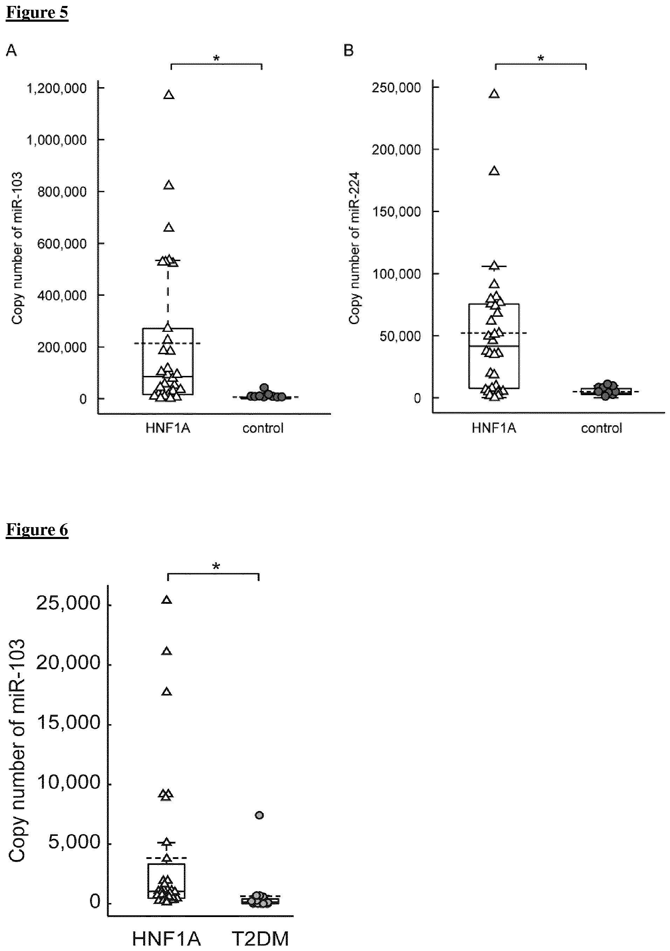

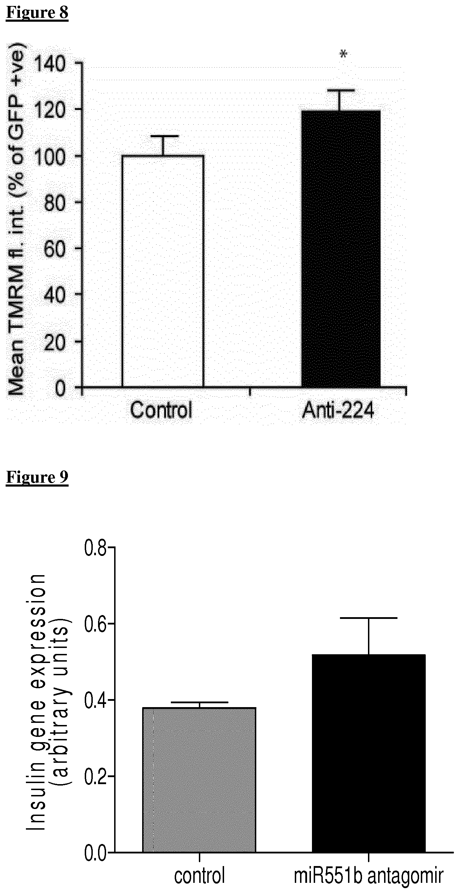

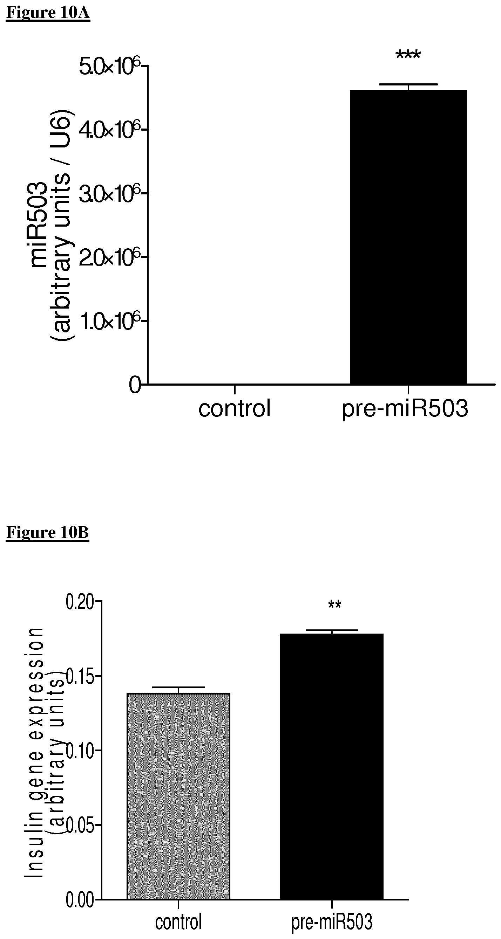

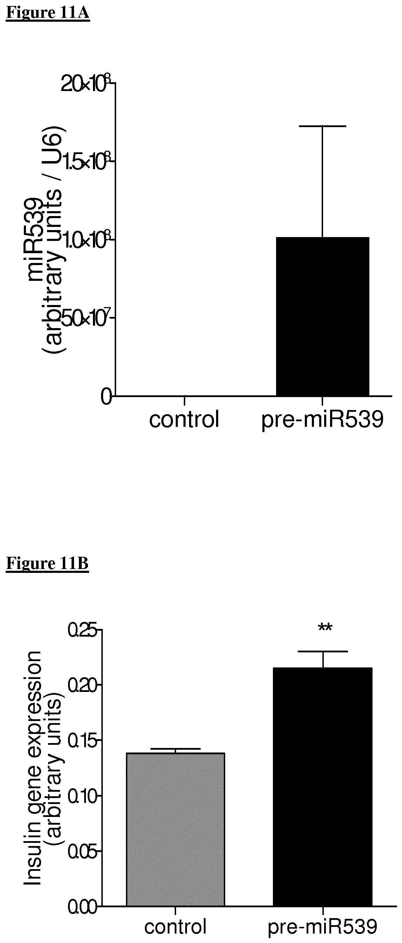

The Applicant has identified a number of micro RNA (miR) molecules that are regulated by loss-of-function mutations of HNF1A associated with HNF1A-MODY, are present in human serum and urine, and exhibit a dysregulated expression profile in HNF1A MODY carriers compared with non-carriers. The miR molecules are miR103, miR224, miR551b, miR503, and miR539. In particular:
- miR-103, miR551b, and miR224 were found to be significantly upregulated, and miR503 and miR539 significantly downregulated, in a cell model of HNF1A-MODY (Fig. 1);
- miR103, miR224 and miR551b were found to be upregulated in samples from human HNF1A-MODY carriers compared with non-carriers (Figs. 2 to 5);
- Rat Insulin Promoter (RIP)-Dominant Negative (DN)-HNFIA transgenic mice (a mouse model of HNF-1AMODY) show increased blood glucose, decreased insulin levels as an indicator of decreased beta cell mass, and increased or maintained pancreatic levels of miR-103, miR-224 and miR-551b (despite the decrease in beta cell mass) when compared with non-transgenic mice;
- miR103 was also found to be able to differentiate HNF1A-MODY carriers from body mass index (BMI)-matched type 2 diabetes patients (Fig. 6);
- miR103 was also found to be elevated in other monogenic forms of diabetes associated with a dysregulation of HNF1A-signalling, in particular HNF4A-MODY patients (HNF4A acts upstream of HNF1A) (Fig. 4);
- Inhibition of miR-224 function in beta-cell derived INS-1 insulinoma cells increased mitochondrial membrane potential indicative of increased mitochondrial function (Fig. 8);
- Delivery of pre-miR-503 or pre-miR-539 to beta-cell-derived INS-1 insulinoma cells increased insulin gene expression (Fig. 10 and 11); and
- Inhibition of miR551bin beta-cell-derived INS-1 insulinoma cells increased insulin gene expression (Figure 9).

Thus, in a first aspect, the invention also relates to a method or assay for determining whether an individual is a HNF1A-MODY carrier or has a defect in HNF1A-signalling (such as HNF4A-MODY patients), comprising a step of assaying a biological sample from the individual to:
(a) detect increased abundance of a micro RNA molecule selected from miR103, miR551b, or miR224; and/or
(b) detect decreased abundance of a micro RNA molecule selected from miR503, and miR539,
wherein increased abundance of one or more of miR103, miR551b, or miR224 and/or decreased abundance of one or more of miR503 and miR539, indicates that the individual is a HNF1A-MODY carrier,or in which increased abundance of one or more of miR103, miR551b or miR224 indicates that the individual is a diabetes patient with a defect in HNF1A signalling.

In particular, the invention relates to a method or assay for determining whether an individual is a HNF1A-MODY carrier or a diabetes patient having a defect in HNF1A signalling, comprising a step of assaying a biological sample from the individual to detect increased abundance of a micro RNA molecule selected from miR103 or miR224, wherein increased abundance of one or more of miR103 or miR224 indicates that the individual is a HNF1A-MODY carrier or a diabetes patient having a defect in HNF1A signalling.

The invention also relates to a method for distinguishing between a HNF1A-MODY carrier and a body mass index (BMI)-matched Type-2 diabetes patient, the method comprising a step of assaying a biological sample from the individual to detect an abundance of miR103, wherein the abundance of miR-103 is lower in a type-2 diabetes patient compared to a HNF1A-MODY carrier.

In another aspect, the invention relates to (a) an inhibitor of miR551b, (in mature, primary or precursor form) and/or (b) miR503 or miR539,(in a mature, primary or precursor form or a mimetic thereof)..for use in a method for the treatment of a metabolic disorder in a patient, wherein the metabolic disorder is characterised by a deficiency in insulin secretion selected from HNF1A-MODY (MODY3); HNF4A-MODY (MODY1); and Type-2 diabetes.

The use in the method of treatment may be a symptomatic therapy, including treatment or prevention of a symptom selected from elevated blood sugar levels, elevated gluconeogenesis, insulin insensitivity, insulin resistance, reduced glucose tolerance, low or reduced insulin levels.

A method for decreasing blood glucose level in a patient, comprises a step of treating the patient with (a) an inhibitor of miR551b, or miR224 and/or (b) miR503 or miR539 in a mature, primary or precursor form or a mimetic thereof.

A method for preventing or delaying onset of an increase in blood glucose level in a patient, comprises a step of treating the patient with (a) an inhibitor of miR551b, or miR224 and/or (b) miR503 or miR539 in a mature, primary or precursor form or a mimetic thereof.

The disclosure relates to a method for decreasing gluconeogenesis in a patient, comprising a step of treating the patient with (a) an inhibitor of miR551b, and/or (b) miR503 or miR539 in a mature, primary or precursor form or a mimetic thereof.

The disclosure relates to a method for improving insulin sensitivity in a patient, comprising a step of treating the patient with (a) an inhibitor of miR551b, and/or (b) miR503 or miR539 in a mature, primary or precursor form or a mimetic thereof.

The disclosure relates to a method for preventing or delaying onset of insulin resistance in a patient, comprising a step of treating the patient with (a) an inhibitor of miR551b, and/or (b) miR503 or miR539 in a mature, primary or precursor form or a mimetic thereof.

The disclosure relates to a method for improving glucose tolerance in a patient, comprising a step of treating the patient with (a) an inhibitor of miR551b, o and/or (b) miR503 or miR539 in a mature, primary or precursor form or a mimetic thereof.

The disclosure relates to a method for increasing insulin levels in a patient, comprising a step of treating the patient with (a) an inhibitor of miR551b, and/or (b) miR503 or miR539 in a mature, primary or precursor form or a mimetic thereof.

The disclosure relates to a method for decreasing plasma cholesterol level in a patient, comprising a step of treating the patient with (a) an inhibitor of miR551b, and/or (b) miR503 or miR539 in a mature, primary or precursor form or a mimetic thereof.
The invention also relates to the use of (a) an inhibitor of miR103, miR551b (in mature, primary or precursor form), for example an antagomir of miR103, miR551b,, or (b) miR503 or miR539 (in mature, primary or precursor form or a mimetic thereof), as a medicament.

The invention also relates to a pharmaceutical composition comprising an active agent and a pharmaceutically acceptable carrier, in which the active agent is selected from (a) an inhibitor of miR103, miR551b, (in mature, primary or precursor form), for example an antagomir of miR103, or miR551b, and/or (b) miR503 and/or miR539 (in mature, primary or precursor form or a mimetic thereof).
In an embodiment, the method for the treatment of a metabolic disorder comprises an initialstep of identifying a patient suitable for treatment, wherein the step of identifying a patient suitable for treatment comprises the steps of:
(a) detecting increased abundance of a micro RNA molecule selected from miR103, miR551b, or miR224; or
(b) detecting decreased abundance of a micro RNA molecule selected from miR503, and miR539,
wherein detection of increased abundance of one or more of miR103, miR551b, or miR224, or detection of decreased abundance of one or more of miR503 and miR539, indicates that the individual is suitable for treatment.

In one embodiment, the metabolic disorder is selected from diabetes (for example, MODY, Type 1 or Type 2 diabetes) and the suitable medicament is selected from sulfonylureas, metformin, glitazones, GLP-1 analogues, DPP-IV inhibitors, insulin and insulin analogues, statins, other lipid- and cholesterol lowering agents, ACE and AT inhibitors and other anti-hypertensive agents.

In one preferred embodiment, the metabolic disorder is MODY and the suitable medicament is sulfonylureas.

Thus, for example, when increased abundance of miR224 is detected in the patient, the patient will be treated with an inhibitor of miR224. Likewise, if decreased abundance of miR503 is detected in the patient, the patient is treated with miR503.

Typically, the suitable medication is a pharmaceutical composition of the invention.

The invention also relates to a method for determining a suitable treatment for an individual with a metabolic disorder, especially an individual with HNF1A-MODY; the method comprising a step of detecting increased abundance of a micro RNA molecule selected from miR224, miR551b or miR103,
wherein detection of increased abundance of one or more of miR224, miR551b or miR103 indicates that the individual is suitable for treatment with a therapy that stimulates insulin production or insulin secretion.

A system for obtaining data from at least one test sample obtained from at least one individual comprises
(a) a determination module configured to receive at least one test sample (i.e. serum or whole blood) and perform at least one test analysis on the test sample to detect increased abundance of a micro RNA molecule selected from miR103, miR551b, or miR224, or detecting decreased abundance of a micro RNA molecule selected from miR503, and miR539,
(b) optionally, a storage system for storing data relating to the abundance of the miR molecules generated by the determination system; and
(c) a display module for displaying a content based in part on the data output from said determination module, wherein the content comprises a signal indicative of the presence or absence of increased abundance of miR103, miR551b, or miR224, or presence or absence of decreased abundance of miR503 or miR539.

Preferably, the determination system comprises means for measuring the level of a micro RNA molecule and then comparing the measured level with a reference level. The reference level may be a level of the same micro RNA from a HNF1A-MODY non-carrier, typically from the same type of sample. In one embodiment, the determination module comprises means for measuring the level of a micro RNA, for example one or more or miR103, miR551b, miR224, miR503 or miR539, from a control or reference sample, for example a biological sample from an individual known to be a HNF1A-MODY non-carrier.

Ideally, the determination system comprises a PCR apparatus.

The system is for determining whether an individual is a HNF1A-MODY carrier. Suitably, the display module is adapted to display a content comprising a signal indicative of whether the individual is a HNF1A-MODY carrier or non-carrier.

A system for determining whether a patient is a HNF1A non-carrier comprises
(a) a determination module configured to receive at least one test sample (i.e. serum or whole blood) and perform at least one test analysis on the test sample to detect the abundance of one or more micro RNA molecule selected from miR103, miR551b, miR224, miR503, and miR539,
(b) optionally, a storage system for storing data relating to the abundance of the miR molecules generated by the determination system;
(c) a comparison module for comparing the detected abundance of the micro RNA molecule selected from miR103, miR551b, miR224, miR503, and miR539 with a reference abundance for the same micro RNA molecule
(d) a display module for displaying a content based in part on the data output from said determination module, wherein the content comprises a signal indicative of the presence or absence of increased abundance of miR103, miR551b, or miR224, or presence or absence of decreased abundance of miR503 or miR539.

The system is for determining a suitable treatment for an individual with a metabolic disorder, especially an individual with MODY; HNF1A-MODY; pre-diabetes, or diabetes (including Type-1 diabetes or Type-2 diabetes). Suitably, the comparison module is adapted to detect increased abundance of miR224, and the display module is adapted to displaying a content based in part on the data output from said determination module, wherein the content comprises (a) a signal indicative of increased abundance of miR224 and/or (b) a content comprising a signal indicative of whether the individual is suitable for treatment with a therapy that stimulates insulin production or insulin secretion.

A system for determining a suitable treatment for an individual with a metabolic disorder, especially an individual with MODY; HNF1A-MODY; pre-diabetes; diabetes (including Type-1 diabetes or Type-2 diabetes), or another condition involving dysregulated insulin production, comprises:
(a) a determination module configured to receive at least one test sample (i.e. serum or whole blood) and perform at least one test analysis on the test sample to detect the abundance of one or more micro RNA molecules selected from miR103, miR551b, miR224, miR503, and miR539,
(b) optionally, a storage system for storing data relating to the abundance of the miR molecules generated by the determination system;
(c) a comparison module for comparing the detected increased abundance of the micro RNA molecule selected from miR224 with a reference abundance for the same micro RNA molecule
(d) a display module for displaying a content based in part on the data output from said determination module, wherein the content comprises (a) a signal indicative of increased abundance of miR224 and/or (b) a content comprising a signal indicative of whether the individual is suitable for treatment with a therapy that stimulates insulin production or insulin secretion.

### Definitions

In this specification, the term "MODY" refers to Maturity Onset Diabetes of the Young, a autosomal dominant form of non-insulin-dependent diabetes affecting young people with a positive family history or with *de novo* mutations. It is also referred to as a monogenic form of diabetes. There are three predominant forms of MODY, namely HNF1A-MODY (MODY3 - caused by mutations of the HNF1alpha gene), HNF4-MODY (MODY1 - caused by loss of function mutations in the HNF4alpha gene), and GCK-MODY (MODY2 - caused by one or more mutations in the GCK gene).

In this specification, the term "increased abundance" as applied to a miRNA molecule should be understood to means significant upregulation of the miRNA molecule compared to the level of the same miRNA molecule in a HNF1A-MODY non-carrier, preferably a HNF1A-MODY non-carrier family control.

Likewise, the term "decreased abundance" as applied to a miRNA molecule should be understood to means significant downregulation of the miRNA molecule compared to the level of the same miRNA molecule in a HNF1A-MODY non-carrier, preferably a HNF1A-MODY non-carrier family control. Methods for determining increased or decreased abundance will be known to a person skilled in the art and include quantitative PCT using a suitable control, for example a sample from a HNF1A-MODY non-carrier.

In this specification, the term "individual having a defect in HNF1A signalling" or "patient having a defect in HNF1A signalling" should be understood to mean a patient having another monogenic forms of diabetes associated with a dysregulation of HNF1A-signalling, for example a patient having HNF4-MODY.

The term "miR103" should be understood to mean hsa-miR-103 in any form, for example mature, primary or precursor sequences. Preferably, the term means mature hsa-miR-103 having the sequence of SEQUENCE ID NO: 1 (Table 4). In one embodiment, the term includes paralogs or functional variants of hsa-miR-103 having at least 80%, 85%, 90% or 95% sequence identity with hsa-miR-103.

The term "miR551b" should be understood to mean hsa-miR-551b in any form, for example mature, primary or precursor sequences. Preferably, the term means mature hsa-miR-551b having the sequence of SEQUENCE ID NO: 2 (Table 4). In one embodiment, the term includes paralogs or variants of hsa-miR-551b having at least 80%, 85%, 90%, or 95% sequence identity with hsa-miR-551b.

The term "miR224" should be understood to mean hsa-miR-224 in any form, for example mature, primary or precursor sequences. Preferably, the term means mature hsa-miR-224 having the sequence of SEQUENCE ID NO: 3 (Table 4). In one embodiment, the term includes paralogs or variants of hsa-miR-224 having at least 90%, 85%, 90% or 95% sequence identity with hsa-miR-224.

The term "miR503" should be understood to mean hsa-miR-503 in any form, for example mature, primary or precursor sequences. Preferably, the term means mature hsa-miR-503 having the sequence of SEQUENCE ID NO: 4 (Table 4). In one embodiment, the term includes paralogs of hsa-miR-503 having at least 80%, 85%, 90% or 95% sequence identity with hsa-miR-503.

The term "miR539" should be understood to mean hsa-miR-539 in any form, for example mature, primary or precursor sequences. Preferably, the term means mature hsa-miR-539-3p having the sequence of SEQUENCE ID NO: 5 or hsa-miR-539-5p having the sequence of SEQUENCE ID NO: 12 (Table 4). In one embodiment, the term includes paralogs of hsa-miR-539 having at least 80%, 85%, 90%, or 95% sequence identity with hsa-miR-509.

The term "variant" as applied to a reference micro RNA molecule should be understood to mean a microRNA molecules that has at least 80%, 85%, 90% and ideally 95% sequence identity with the reference micro RNA molecule. The variants may include modified oligonucleotides. Example of possible modification are provided in paragraphs 47-85 of US20050227934 (the complete contents of which are incorporated herein by reference).

The term "biological sample" should be understood to mean any sample from a human being, for example whole blood, serum, plasma, urine, saliva, cerebrospinal fluid. Preferably the sample is blood or blood derived, for example serum, or urine.

The term "assaying" should be understood to mean quantitative detection of one or more of the miRNA molecules in the sample. Suitable methods will be known to a person skilled in the art, and include quantitative PCR (qPCR) and hybridisation assays, or RNA sequencing.

In the specification, the term "inhibitor" as applied to a micro RNA molecule should be understood to mean a molecule capable of inhibiting or preventing the target micro RNA carrying out its function(s). The inhibitor molecule may inhibit the target micro RNA in a mature, primary or precursor form. Generally, the inhibition should be understood to include direct inhibition in which a molecule binds to the target miR and directly inhibits its activity (for example, a low molecular weight inhibitor or a binding partner such as an antibody or antibody fragment) and indirect inhibition in which, for example, expression of the miRNA molecule is modulated by suitable means including for example use of repressors or small interfering RNA molecules. Inhibition of miR function should be understood to encompass administering any molecule which directly or indirectly inhibits the mature form as well as molecules which target the primary or precursor forms of the target miR, as well as any paralogues. The term should also include administering molecules which interfere with the miR function by enhancing expression of its target mRNA transcripts, or by means of antisense targeting. The term "inhibitor" should preferably be understood to mean any agent which may inhibit, ideally specifically inhibit, the expression of or activity miR-134. Suitable agents include antagomirs, antisense molecules, small hairpin RNA molecules, small interfering RNA molecules, microRNA sponges, tiny seed-targeting locked nucleic acid (LNA) oligonucleotides, decoy oligonucleotides, aptamers, ribozymes, or antibodies that specifically recognize DNA:RNA heteroduplexes. Small hairpin RNA (shRNA) molecules are short RNA molecules having a small hairpin loop in their tertiary structure that may be employed to silence genes. The design of shRNA molecules capable of inhibiting a target miR will be apparent to those skilled in the field of shRNA molecule design. As an alternative, the level of miR expression can be modulated using antisense or ribozyme approaches to inhibit or prevent miR activity, or triple helix approaches to inhibit transcription of miR. In a preferred embodiment, the inhibitor is a molecule or complex that comprises an oligonucleotide that is at least partially complementary (or anti-sense_) to the target micro RNA molecule. The inhibitor oligonucleotide is typically 7-30 linked nucleosides that is at least 85%, 90%, 95%, 96%, 97%, 98%, 99% or fully, complementary to the sequence of the target micro RNA is a mature form. The inhibitor oligonucleotide may be modified. Example of possible modification are provided in paragraphs 38-39 of US2012/0122959 (the complete contents of which are incorporated herein by reference). miRNA inhibitors are sold by the company Ambion under the name MIRvANA™.

In the specification, the term "antagomir" should be understood to mean a novel class of chemically engineered oligonucleotides. Antagomirs are used to silence endogenous microRNA. An antagomir is a small synthetic oligonucleotide that is complementary to the specific miRNA target with either mispairing at the cleavage site of Arganoute 2 (Ago2) or some sort of base modification to inhibit Ago2 cleavage. Usually, antagomirs have some sort of modification, such as 2' methoxy groups, 3'-cholesterol groups, phosphorothioates, to make it more resistant to degradation. It is believed that antagomirs inhibit by irreversibly binding the miRNA.

In this specification, the term "mimic" as applied to a micro RNA molecule should be understood to mean functional variants of the micro RNA molecule, or small, chemically modified double-stranded RNAs that mimic endogenous miRNAs and enable miRNA functional analysis by up-regulation of miRNA activity. The are sold by the company Ambion under the name MIRvANA™.

Various delivery systems are known and can be used to administer a therapeutic of the invention, *e.g.,* intra-nasally Methods of introduction include but are not limited to intradermal, intramuscular, intraperitoneal, intravenous, subcutaneous, intranasal, epidural, intranasal, intracerebral, and oral routes. The compositions may be administered by any convenient route, for example by infusion or bolus injection, by absorption through epithelial or mucocutaneous linings (*e.g.,* oral mucosa, rectal and intestinal mucosa, *etc.*) and may be administered together with other biologically active agents. Administration can be systemic or local. In addition, it may be desirable to introduce the compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.,* by use of an inhaler or nebulizer, and formulation with an aerosolizing agent.

It may be desirable to administer the compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

The present invention also provides pharmaceutical compositions. Such compositions comprise a therapeutically effective amount of the therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In the case of MODY, the term "therapeutically effective amount" should be taken to mean an amount of therapeutic which results in a clinically significant inhibition, amelioration or reversal of symptoms of MODY, or in the case of type-2 or type 1 diabetes, clinically significant inhibition, amelioration or reversal of development symptoms of type-2 diabetes.

The term "carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The term "suitable medication" as applied to MODY means sulfonylureas, metformin, glitazones, GLP-1 analogues, DPP-IV inhibitors, insulin and insulin analogues, statins, other lipid- and cholesterol lowering agents, ACE and AT inhibitors and other anti-hypertensive agents, diet and exercise only.

The term "suitable medication" as applied to Type-2 and type 1 diabetes means sulfonylureas, metformin, glitazones, GLP-1 analogues, DPP-IV inhibitors, insulin and insulin analogues, statins, other lipid- and cholesterol lowering agents, ACE and AT inhibitors and other anti-hypertensive agents, diet and exercise only.

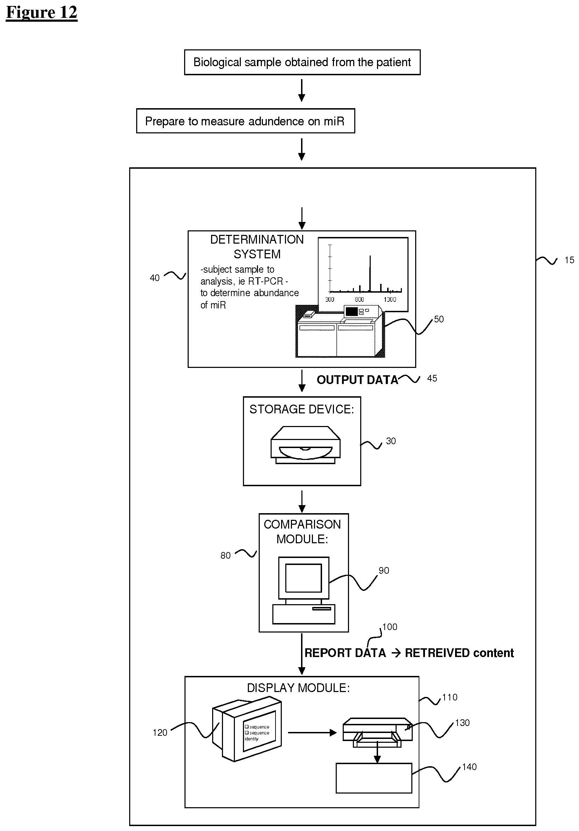

An example of a systems (and computer readable media for causing computer systems) to perform a method for detecting and/or identifying that a patient is a HNF1A-MODY carrier or non-carrier, or determining a suitable treatment for an individual with a metabolic condition/disease is illustrated in Figure 12.

Embodiments of the system can be described through functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions. The functional modules of certain embodiments of the invention include at minimum a determination module #40, a storage module #30, optionally, a comparison module #80, and a display module #110. The functional modules can be executed on one, or multiple, computers, or by using one, or multiple, computer networks. The determination system has computer executable instructions to provide e.g., sequence information in computer readable form.

The storage module #30 which can be any available tangible media that can be accessed by a computer. The storage module (i.e. computer readable storage media) includes volatile and nonvolatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (erasable programmable read only memory), EEPROM (electrically erasable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable storage media may define instructions, for example, as part of one or more programs, that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable storage media on which such instructions are embodied may reside on one or more of the components of either of a system, or a computer readable storage medium described herein, may be distributed across one or more of such components.

The computer-readable storage media may be transportable such that the instructions stored thereon can be loaded onto any computer resource to implement the aspects of the present invention discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects of the present invention. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The determination module #40, can comprise any system for detecting increased or decreased abundance of the relevant micro RNA molecules. Standard procedures such as quantitative PCR can be used. Additionally one can determine other factors relevant to diagnosis of HNF1A-MODY, for example C-reactive protein. These factors can be used in conjunction with levels of relevant micro RNA molecules..The information determined in the determination system can be read by the storage device #30. As used herein the "storage device" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device is adapted or configured for having recorded thereon nucleic acid sequence information. Such information may be provided in digital form that can be transmitted and read electronically, e.g., via the Internet, on diskette, via USB (universal serial bus) or via any other suitable mode of communication.

As used herein, "stored" refers to a process for encoding information on the storage device. Those skilled in the art can readily adopt any of the presently known methods for recording information on known media to generate manufactures comprising information relating to micro RNA levels.

The reference data stored in the storage device to be read by the comparison module is compared for the purpose of detecting increased or decreased abundance of one or more specific micro RNA molecules compared with a reference abundance.

The "comparison module" #80 can use a variety of available software programs and formats for the comparison operative to compare micro RNA abundance determined in the determination system to reference samples and/or stored reference data. In one embodiment, the comparison module is configured to use pattern recognition techniques to compare information from one or more entries to one or more reference data patterns. The comparison module may be configured using existing commercially-available or freely-available software for comparing patterns, and may be optimized for particular data comparisons that are conducted. The comparison module provides computer readable information related to the abundance of one or more micro RNA molecules in a sample relative to a reference abundance (i.e. relative to abundance in a HNF1A-MODY non-carrier).

The comparison module, or any other module of the invention, may include an operating system (e.g., UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (e.g., the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module provides a computer readable comparison result that can be processed in computer readable form by predefined criteria, or criteria defined by a user, to provide a content based in part on the comparison result that may be stored and output as requested by a user using a display module #110.

In one embodiment the content based on the comparison result is displayed on a computer monitor #120. In one embodiment of the invention, the content based on the comparison result is displayed through printable media #130, #140. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

A World Wide Web browser is used for providing a user interface for display of the content based on the comparison result. It should be understood that other modules of the invention can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

The methods described herein therefore provide for systems (and computer readable media for causing computer systems) to perform methods as described in the Statements of Invention above, for example (a) methods of identifying an individual that is a HNF1A-MODT carrier.

Systems and computer readable media described herein are merely illustrative embodiments of the invention for performing methods of diagnosis in an individual, and are not intended to limit the scope of the invention. Variations of the systems and computer readable media described herein are possible and are intended to fall within the scope of the invention.

The modules of the machine, or those used in the computer readable medium, may assume numerous configurations. For example, function may be provided on a single machine or distributed over multiple machines.

### Brief Description of the Figures

**Figure 1****.** *Identification of miR-103miR-224, miR-551b, miR-503 and miR-539 to be differentially expressed in INS-1 inducibly expressing Pro291fsinsC-HNF1A.* **A.** Time course of Pro291fsinC-HNF1A mRNA induction in INS-1 cells. Inducible INS-1 cells were treated with doxycycline at a concentration of 500 ng/ml for 0, 24 h and 48 h, RNA was isolated and Pro291fsinC-HNF1A mRNA levels determined by absolute RT-qPCR. Experiments were carried out in triplicate (*p<0.05). **B.** Identification of miRNA regulated by Pro291fsinC-HNF1A using rodent miRNA array plates. The array was carried out using biological duplicates after 0, 24 h and 48 h of doxycycline induction. Data is represented as RQ of each biological sample at both 24 h and 48 h time-points relative to 0 h time-point which was set to 1. C. Validation of miRNA expression changes. Pro291fsinC-HNF1A expression was induced as described above, and a TaqMan PCR was carried out for the expression of miR-103 and miR-224 . ΔCt values were calculated by subtracting the Ct value for miR-29a (control) from the corresponding Ct value for each miRNA. Experiments were carried out in triplicate (*=p<0.05).
**Figure 2****.** *HNF1A-MODY carriers show elevated serum levels of miR-103 and miR-224.* miR-103 (black) is significantly higher in HNF1A-MODY than in MODY-negative family controls (median=1030, IQR=458-3318 vs. median=14, IQR=0-126, p=0.00001). Serum levels of miR-224 (grey) are also elevated in HNF1A-MODY compared to family controls (median=2610, IQR=9-18475 vs. median=33, IQR=23-46, p=0.0467).
**Figure 3****.** *HNF1A-MODY carriers show elevated serum levels of miR-551b.* Quantification of miR551b expression in human serum of MODYnegative family controls (n=13) and MODY3 patients (n=23) by *TaqMan qRT-PCR.* Ct values were converted to absolute number of copies/reaction for 3µL serum by using a dilution series of known input quantities of synthetic target miRNA run simultaneously (on the same plate) as the experimental samples. (n= 13+23; Means ± SEM).
**Figure 4****:** *miR-103 is also elevated in HNF-4A-MODY patients.* Absolute miR-103 mRNA levels in serum from n=10 MODY-negative family members (Control), n= 9 HNF4A-MODY (MODY1) carriers, and n=30 HNF1A-MODY (MODY3) carriers. Difference between Control and HNF4A-MODY: p<0.05 (Kruskal Wallis H Test and Mann-Whitney U test).
**Figure 5****.** *HNF1A-MODY carriers show elevated urine levels of miR-103 and miR-224.* Urine levels of miR-103 and mir-224 are detectable in HNF1A-MODY carriers when compared to MODY-negative family controls. Data are from n=30 HNF1A-MODY patients and n=9 MODY-negative family controls. P values: miR-103: p =0.00034. miR-224: p = 0.00183 (U-test):
**Figure 6****.** *miR-103 can discriminate Body Mass Index- (BMI) and HbA1C-matched T2DM patients from HNF1A-MODY patients.* Boxplot showing median and inter quartile range (IQR) of the two patient cohorts (number of Type-2 diabetes patients studied n=17). The dashed line indicates the mean of miRNA copy numbers. Serum levels of miR-103 are significantly lower in T2DM patients than in HNF1A-MODY (○ median=129, IQR=8-399 vs. ▲median=1030, IQR=458-3318, *** p=0.00003).

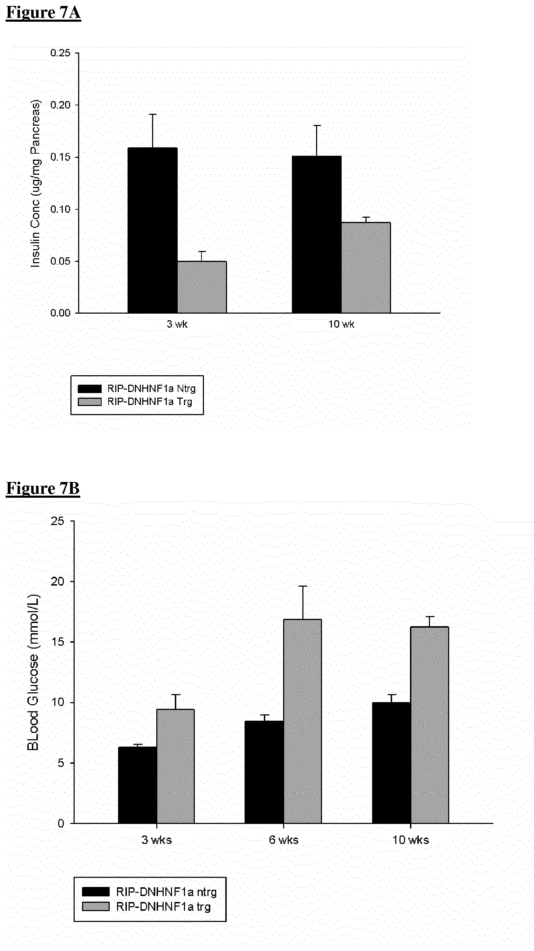

**Figure 7A****.** *RIP-DN-HNF1A transgenic mice show a reduction in pancreatic insulin content*Pancreatic insulin content as determined from 3 week and 10 week-old male animals. Pancreata were removed, homogenised and treated as described in Methods. Insulin content in acid-ethanol supernatant was determined with mouse Insulin ELISA (Mercodia AB). Statistical analysis was carried out in SPSS and data expressed as mean insulin concentration ug/mg pancreas ± SEM. Difference between tg and ntg mice: P<0.05 (ANOVA and Tukey test). n=3 per genotype.
**Figure 7B****.** *RIP-DN-HNF1A transgenic mice develop diabetes* Homeostatic blood glucose levels were measured in male transgenic (tg) and non-transgenic (ntg) animals at 3, 6 and 10 week timepoints. Difference between tg and non-tg mice: P<0.05 (ANOVA and Tukey test).Data are expressed as mean +/- SEM for n=5 ntg and n=9 tg mice.

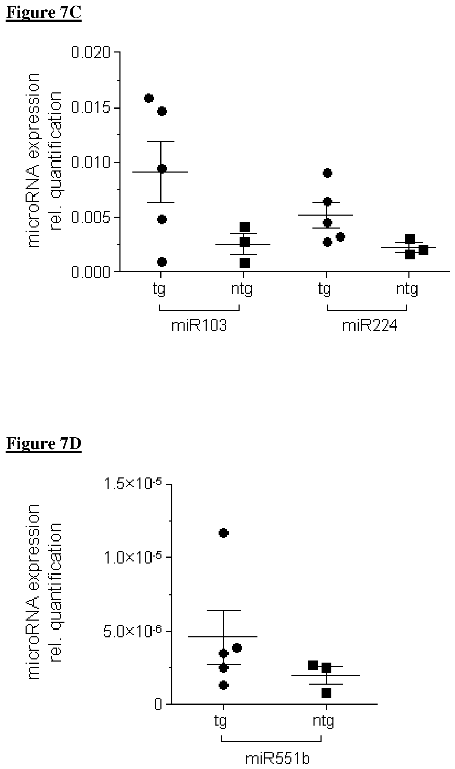

**Figure 7C and 7D****.** *microRNA analysis in mouse pancreas* Pancreata from male RIP-DNHNF1A transgenic and control non-transgenic mice were harvested and immediately snap frozen in liquid nitrogen. RNA enriched in miRNAs was prepared from pancreas samples and analysed for miRNA expression levels. The data was normalised to the endogenous control U6snRNA. Data were analyzed by the relative quantification (ΔΔCt) method. Data are shown as means +/- SEM.
**Figure 8****:** *Inhibition of miR-224 increases TMRM fluorescence intensity in INS-1 cells* Cells were cotransfected with antagomir-224 and GFP as described in the methods section. TMRM fluorescence intensity was measured in -300 GFP positive cells per well and data represents the mean ± SEM of the mean fluorescence intensity of cell populations from n=4 wells per treatment, expressed as a percentage of the control value. *p<0.05 compared to control (t-test).
**Figure 9****.** *Inhibition of miR-551b increases intracellular insulin content in INS-1 cells* Measurement of insulin mRNA expression using relative qPCR in native INS-1 cells transfected with transfected with control Inhibitor and miR551b Inhibitor. In relative quantification the expression of insulin mRNA was measured with respect to the reference gene β-actin which was expressed constitutively . (n=4; t-test vs. control: not significant)
**Figure 10****:** *Transfection of native INS-1 cells with miR-503 increases insulin production:* (A) Measurement of miR503 in native INS-1 cells transfected with pre-miR-control and pre-miR503. (n=3; t-test vs. control: p<0.001); (B) Measurement of insulin mRNA expression using relative qPCR in native INS-1 cells transfected with transfected with pre-miR-control and pre-miR503. In relative quantification the expression of insulin mRNA was measured with respect to the reference gene β-actin which was expressed constitutively . (n=3: t-test vs. control: p<0.01)
**Figure 11****:** *Transfection of native INS-1 cells with miR-539 increases insulin production:* (A) Measurement of miR539 in native INS-1 cells transfected with pre-miR-control and pre-miR539. (n=3; t-test vs. control: p<0.001); (B) Measurement of insulin mRNA expression using relative qPCR in native INS-1 cells transfected with transfected with pre-miR-control and pre-miR539. In relative quantification the expression of insulin mRNA was measured with respect to the reference gene β-actin which was expressed constitutively . (n=3: t-test vs. control: p<0.01)
**Figure 12** A system for determining whether an individual is a HNF1A-MODY carrier.

### Detailed Description of the Invention

### Research design and methods

### INS-1 cells overexpressing HNF1A in an inducible system.

Rat INS-1 insulinoma cells inducibly expressing the human HNF1A-MODY frameshift mutant Pro291fsinsC-HNF1A under the control of a doxycycline-dependent transcriptional activator have been described previously [3, 9-10, 20]. The Pro291fsinsC-HNF1A mutant has been shown to bind endogenous HNF1A, and to act as a dominant-negative transcription factor *in vitro* [4]. Cells were cultured in RPMI 1640 at 6 mM glucose supplemented with 10% Fetal Bovine Serum (FBS) (PAA, Cölbe, Germany), 2 mmol/l L-glutamine, 1 mmol/l pyruvate, penicillin (100 U/ml), streptomycin (100 µg/ml), 10 mmol/l HEPES (pH 7.4) and 50 µmol/l 2-mercaptoethanol (Sigma, Dublin, Ireland) [21]. Measurement of *Hnfla* induction in INS-1 cells was carried out using absolute qPCR. A *Hnf1a* gene-specific PCR amplicon was prepared as a standard. The calibration curve was created by plotting the threshold cycle (Ct) corresponding to each standard versus their corresponding log number of *Hnfla* standard (expressed as cDNA copy number of the *Hnfla* gene). Normalization was carried out using total RNA values, as previously described [9].

Database analysis was carried out to determine possible gene targets of the experimentally validated, differentially expressed miRNAs. We employed the use of two different target prediction algorithms, miRanda and TargetScan, for our analysis. Gene functional analysis was carried out using DAVID to group putative gene targets into functional signalling KEGG pathways. The number of genes (count) and the percentage of genes from the predicted lists are shown in Table 1. Analysis of putative target genes for miR-103 and miR-503 revealed the insulin signalling pathway to be amongst the top signalling pathways targeted. Analysis of putative target genes for miR-103 also revealed Ca²⁺ signalling. Analysis of putative target genes for miR-224 revealed endocytosis and TGF-beta signalling.

### TaqMan microRNA array

INS-1 cells were cultured in T25 flasks in complete medium with or without 500 ng/ml doxycycline for 24 h or 48 h. Cells were harvested for total RNA using the Qiagen miRNeasy kit (Qiagen, Hilden, Germany) according to manufacturer's protocol. All downstream applications were performed on ice in an RNase free environment to prevent miRNA degradation. Thirty nanograms of total RNA enriched in miRNAs was converted to cDNA using the TaqMan® MicroRNA Reverse Transcription Kit (Roche-Applied Biosystems), and preamplification was carried out on the cDNA using Megaplex PreAmp Primers (Invitrogen). TaqMan® Universal PCR Master Mix (Roche-Applied Biosystems), was added to the samples which were then loaded onto TaqMan® Rodent MicroRNA A+B Cards Set v2.0 (Invitrogen) and run on a 7900HT system using SDS software. Each card contains 3 endogenous rat controls. The data was normalised to the endogenous control MammU6 (MammU6-4395470). The controls U87 (U87-4386735) and Y1 (Y1-4386739) were normalised to the MannU6. Prior to calculating relative expression values, normalization was carried out by subtracting the average endogenous control sample Ct from the individual miRNA Ct values. Relative expression (RQ) of miRNA was calculated using the ΔΔct method; RQ = 2^{-(ΔΔCt)}. P-values were calculated using a t-test assuming two-tailed distribution in two samples of unequal variance.

### Real-time quantitative PCR analysis

INS-1 cells were cultured in 6-well plates in complete medium with or without 500 ng/ml doxycycline for 24 h or 48 h. Cells were harvested for RNA enriched in miRNAs using the Qiagen miRNeasy kit according to the manufacturer's protocol. Reverse transcription was carried out on 30 ng of RNA using the TaqMan® MicroRNA Reverse Transcription Kit, and Taqman probes specific to the individual miRNAs chosen (miR-103, miR-224, and miR-29a). Real-time PCR amplification was performed with the reverse transcription products using TaqMan® 2X Universal PCR Master Mix without UNG Amperase, and miRNA specific Taqman probes (Roche-Applied Biosystems):
hsa-miR-103 (AGCAGCAUUGUACAGGGCUAUGA); SEQ ID NO: 1
hsa-miR-224 (CAAGUCACUAGUGGUUCCGUU); SEQ ID NO: 3
hsa-miR-503 (UAGCAGCGGGAACAGUUCUGCAG); SEQ ID NO; 4
hsa-miR-29a (UAGCACCAUCUGAAAUCGGUUA) SEQ ID NO: 6

As miRNA-29a did not show any change of expression in the miRNA array it was chosen as an internal control for the real-time PCR in INS-1 cells overexpressing the Pro291fsinC-HNF1A mutant. Samples were run on an Applied Biosystems 7500 Real-Time PCR system with an initial denaturation at 95 °C for 10 min, followed by 60 cycles at 95 °C for 15 s and 60 °C for 1 min.

### Prediction of gene targets

Potential gene targets of differentially expressed miRNAs were predicted using the two public databases miRanda (http://www.microrna.org/microrna/home.do) and TargetScan (http://www.targetscan.org/). The gene functional classification tool DAVID (http://david.abcc.ncifcrf.gov/home.jsp) was used to identify the signalling pathways to which the predicted gene targets belonged [22-23].

### Subjects and clinical and laboratory measurements

Subjects with a clinical diagnosis of MODY were recruited from the MODY diabetes clinics in the Mater Misericordiae University Hospital Dublin in Ireland. Sequencing of the *HNF1A* gene was performed by IntegraGen (Bonn, Germany) in 2006-2007 and the Molecular Genetics Laboratory (Exeter, UK) in 2008-2010. Genetically confirmed MODY subjects included 31 cases with *HNF1A* mutations. The subjects with *HNF1A* mutations were from 11 pedigrees and the mutations included L17H (n=1), G207D (n=1), P291finsC (n=14), S352fsdelG (n=9), F426X (n=2), P379T (n=2), and IVS7-6G>A (n=2). The mutations named above have been previously published and are known to co-segregate in families with diabetes [24-25]. There were 10 normoglycaemic BMI-matched HNF1A-MODY negative family members as well as which were available as a control group. All subjects underwent a clinical assessment including a full medical history and physical examination. Details of the subjects' weight, height and blood pressure were recorded. A 75 g OGTT was performed on subjects after a 12-h overnight fast with measurement of glucose, insulin and C-peptide at baseline and at 30 minute intervals for 120 minutes to determine the degree of glucose tolerance and insulin secretory response. In patients with diabetes, oral hypoglycaemic agents were stopped at least 48-h before the OGTT while, in those taking insulin, long-acting insulin therapy was stopped for 24-h and short-acting insulin stopped for 12-h prior to OGTT. The diagnostic criteria for the American Diabetes Association was used to define the degree of glucose tolerance. The oral glucose insulin sensitivity (OGIS) was calculated as previously described [26]. The plasma glucose concentration was measured using Beckman Synchron DXC800 (Beckman Instruments Inc, Brea, USA). HbA_{1c} was determined using high performance liquid chromatography (Menarini HA81-10, Rome, Italy). Insulin and C-peptide were analyzed using Immulite 2000 immunoassay (Siemens Healthcare Diagnostics, Deerfield, IL, USA).

The study was approved by the Research Ethics Committee at the Mater Misericordiae University Hospital Dublin and all subjects gave informed written consent.

### RNA Isolation from serum and absolute qRT-PCR assays

Total RNA enriched with miRNAs was isolated from HNF1A-MODY carriers and MODY-negative, non-diabetic family member sera using the Total RNA Purification Kit (Norgen, Biotek Cooporation, ON, Canada). Reverse transcription was carried out on 10 ng of RNA using the TaqMan® MicroRNA Reverse Transcription Kit, and Taqman probes specific to the individual miRNAs (miR-103 and miR-224). Individual miRNAs from patient serum samples were detected by absolute qPCR. TaqMan assays for human miRNAs hsa-miR-103 and hsa-miR-224 were obtained from Applied Biosystems. Oligoribonucleotides corresponding to the mature sequence of each miRNA were synthesized (Sigma Aldrich) and reverse transcribed to generate a standard curve. The oligoribonucleotides sequences used were: miR-103 mature sequence: AGCAGCAUUGUACAGGGCUAUGA (SEQ ID NO: 1); and miR-224 mature sequence: CAAGUCACUAGUGGUUCCGUU (SEQ ID NO: 3). The standard curve was established using cDNA generated from the 23-bp or 25-bp human miR-103 / miR-224 mature sequence amplicons, respectively. Two microlitres of cDNA were used as templates for PCR using the LightCycler 2.0. The miR-103 and miR-224 amplicons were isolated and purified using QIAquick PCR purification kit (QiaGen). The absolute quantity of the purified amplicons were measured by their absorbance at 260 nm and converted to the number of copies using the molecular weight of DNA. The miR-103 and miR-224 amplicons were diluted to the concentration of 5.98 x 10⁷ / 5.87 x 10⁷ copies per µl (stock solution). When a standard curve was to be established for the real-time quantitative PCR assay, the stock solution was diluted over 7 orders of magnitude (e.g. 5.98 x 10⁷, 5.89 x 10⁶, 5.89 x 10⁵, 5.89 x 10⁴, 5.89 x 10³, 5.89 x 10, and 5.89 x 10¹, copies per 2 µl). All unknown sample concentration had to fall within this range. Samples were run on the LightCycler 2.0 system (Roche) with an initial denaturation at 95 °C for 10 min, followed by 70 cycles at 95 °C for 10 sec (denaturation) 60 °C for 20 sec (annealing) and 72 °C for 1 sec. The data were analysed using LightCycler Software 4.0®. The normalized values (*dC*T) from serum of HNF1A-MODY carriers were compared with serum from MODY-negative, non-diabetic family members.

### Cell culture & transfection

INS-1 rat insulinoma derived cells were cultured in RPMI supplemented with foetal bovine serum (10%) penicillin (100 units/ml), streptomycin (100 µg/ml), and glutamine (2 mM, Sigma Aldrich, Dublin). For experiments, cells were plated in 96 well plate (Corning, New York, USA) at a density of 10,000 cells per well. After 24 h cells were transfected in OptiMEM using Dharmafect Duo transfection reagent as per manufacturer's instructions. Either an antagomir targeting mir 224 or a control antagomir (100 nM, Exiqon, Vedbaek, Denmark) were cotransfected with plasmid encoding GFP (50 ng DNA/well).

### Insulin staining

Following transfection, cells were cultured for 72 h before fixation in paraformaldehyde (4% for 15 min). Polyclonal anti-swine Insulin antibody raised in guinea pig (Dakocytomation, Stockport, UK) was diluted 1:100 in H₂O with 5% goat serum and 0.1% Triton T100 (Sigma Aldrich, Dublin) and 100 µL was added to each well for 1 h at room temperature. Each well was then washed three times with 100 µL Hank's Balanced Salt Solution (Sigma Aldrich, Dublin) and anti-guinea pig IgG (H+L) raised in goat with conjugated Alexa Fluor® 568 (diluted 1:100 in H₂O with 5% goat serum, Invitrogen, Dublin) was added for 1 h at room temperature.

### Flow cytometry

Live cells were stained with TMRM (20 nM) or fixed and stained insulin antibody as described above. Cells were then trypsinised and single-cell fluorescence intensity measured using a BD LSR II flow cytometer. GFP was measured using a blue (488 nm) laser with both 505LP and 525/50 nm emission filters and GFP positive cells were gated and taken as the transfected population. The yellow/green (561 nm) was used for TMRM or Alexa Fluor® 568 with 605/40 nm emission filter. Data was exported as FCS files and analysis was carried out using Cyflogic software.

### RIP-DN-HNF1a Blood Glucose Measurement

Homeostatic blood glucose levels were measured in male and female animals at 3, 6 and 10 week timepoints using Bayer CONTOUR® Blood Glucose Meter. A small drop of blood (∼0.6ul) from the tail vein was collected and placed on a glucometer test strip. After 45 second developing time, the baseline blood glucose value is recorded (mmol/L) and the mouse returned to its cage.

### RIP-DN-HNF1a Pancreatic Insulin Content

Pancreatic insulin content as determined from 3wk and 10wk male animals. Pancreata were removed and immediately frozen in liquid nitrogen, weighed and incubated O/N in Acid-EtOH (1.5%HCl in 70% Ethanol) at -20°C. Samples were then homogenised and incubated O/N at -20°C. Samples were centrifuged 15 min 2000rpm at 4°C, supernatant removed and neutralised with 1:1 volume TRIS (1M pH7.5). Insulin content in acid-ethanol supernatant was determined with mouse Insulin ELISA (Mercodia AB). Statistical analysis was carried out in SPSS and data expressed as mean insulin concentration ug/mg pancreas ± SEM. n=3 per genotype.

### microRNA analysis in mouse pancreas

Newborn male mice were used for the microRNA analysis. Mouse pancreases were harvested and immediately snap frozen in liquid nitrogen. RNA enriched in miRNAs was prepared from pancreas samples using the Qiagen miRNeasy kit (Qiagen, Hilden, Germany). according to the manufacturers' protocol. For Real-time quantitative PCR analysis 500ng of total RNA enriched in miRNAs was reverse-transcripted using the TaqMan MicroRNA Reverse Transcription Kit (Life technologies, Carlsbad, USA) and individual Taqman primer (Life technologies, Carlsbad USA): hsa-miR-103_AGCAGCAUUGUACAGGGCUAUGA (Assay ID000439) SEQ ID 1, mmu-miR-224_UAAGUCACUAGUGGUUCCGUU (Assay ID002553) SEQ ID 13 hsa-miR551b_GCGACCCAUACUUGGUUUCAG (Assay ID001535) SEQ ID 2 were used for PCR according to the manufacturer's instructions. Real-time PCR amplification was performed with the reverse transcription products using TaqMan 2×Universal PCR Master Mix without UNG Amperase, and miRNA-specific TaqMan probes. The data was normalised to the endogenous control U6snRNA (Assay ID_001973). Data were analyzed by the relative quantification (ΔΔCt) method.

### INS-1 cells and cell culture

Rat INS-1 insulinoma cells overexpressing the gene encoding wild-type (WT) HNF-1alpha or a DN mutant of HNF-1alpha (DN-HNF-1alpha) under control of a doxycycline-dependent transcriptional activator were cultured in RPMI 1640 at 11.1 mmol/l glucose supplemented with 10% fetal bovine serum (FBS_PAA, Cölbe, Germany), 2 mM L-glutamine, 1 mM sodium pyruvate, 100 U/ml penicillin, 100 µg/ml streptomycin, 10 mM HEPES and 50 µM 2-mercaptoethano (Asfari et al. 1992). INS-1 Cells were maintained at 37°C in a humidified incubator gassed with 5% CO₂.

### Plasmid design and generation

To generate the rno-miR224 expression plasmid which co-expresses EGFP, the full length EGFP sequence was subcloned from the pEGFP-N1 (Clontech) into the pLVX-puro vector (Clontech) using EcoRI and Xbal restriction sites to generate pLVX-EGFP-puro. The rat miR-224 pre-miR sequence was identified from the Ensembl database and a 302 bp sequence containing 110 bp 5' to the pre-miR sequence was synthesised by gene synthesis (Eurofins) and subcloned into the Xbal site of the pLVX-EGFP vector to generate pLVX-EGFP-rno-miR-224. All constructs were verified by sequencing.

### Transfection with microRNA224 plasmid

One day before transfection, INS-1 cells were dispersed with trypsin-EDTA solution. For transient transfection INS-1 cells were grown in a 6-well plate at the destiny of 2x10⁵ cells per well. The 224plasmid (1 µg/well) was incubated with 4 µl/well TurboFect (Thermo Fisher Scientific, Waltham, Massachusetts, USA) dilute in 195µl OptiMEM (Life technologies, Carlsbad, CA, USA) for each 6-well and incubated for 20 min at room temperature. While lipid/DNA complexes were forming, the cell culture medium was removed and replaced with 800 µl OptiMEM. DNA/transfection reagent mix was then added to the cells. INS-1 cells were exposed to the transfection cocktail for 4h. The transfection cocktail was then removed and replaced with 2ml normal cell culture medium or culture medium supplemented with 500 ng/ml doxycycline (Sigma, Munich, Germany) for 48 h to induce the production of HNF-1alpha. All Assays were done 24h or 48h after transfection.

### Transfection with miR551b inhibitor (antagomir)

To analyze the function of miR551b in INS-1 cells we used a specific miR551b Inhibitor, which is designed for specific in vitro silencing of miR551b.

One day before transfection, INS-1 cells were dispersed with trypsin-EDTA solution. For transient transfection INS-1 cells were grown in a 6-well plate at the destiny of 2x10⁵ cells per well. Cells were transfected with 75 nM of miRCURY LNA has-miR551b Inhibitor (Sequence 5'-3': TGAAACCAAGTATGGGTCG; (SEQ ID 20) - Exiqon, Vedbaek, Denmark) or with a non-silencing negative control (Sequence 5'-3': GGTAACACGTCTATACGCCCA; (SEQ ID 21) - Exiqon, Vedbaek, Denmark) using DharmaFECT Duo transfection reagent (Thermo Fisher Scientific, Waltham, Massachusetts, USA) according to the manufacturer's instructions. Briefly, 1.5 µl of miRCURY LNA Inhibitor (Stock solution 50 µM) and 4 µl of transfection reagent were diluted with 194.5 µL Opti-MEM (Invitrogen Carlsbad, CA, USA), and incubated for 20 min at room temperature, and then added to the cells. While lipid/DNA complexes were forming, the cell culture medium was removed and replaced with 800 µl OptiMEM. Transfection mix was added to the cells and after 4h the transfection cocktail was replaced with2ml normal cell culture medium or culture medium supplemented with 500 ng/ml doxycycline (Sigma, Munich, Germany) for 48 h to induce the production of HNF-1alpha. All Assays were done 24h or 48h after transfection.

### Transfection with microRNA pre-miRs

To analyze the function of miR503 and miR539 in INS-1 cells we used miRNA mimics. This are small, chemically modified double-stranded RNAs that mimic endogenous miRNAs and enable miRNA functional analysis by up-regulation of miRNA activity. One day before transfection, INS-1 cells were dispersed with trypsin-EDTA solution. For transient transfection INS-1 cells were grown in a 6-well plate at the destiny of 2x10⁵ cells per well. Cells were transfected with 50 nM of mirVana™ miRNA mimics for mmu-miR502 (ID: MC12867; mature miRNA sequence UAGCAGCGGGAACAGUACUGCAG - SEQ ID 14) or has-miR539B (ID: MC11336; mature miRNA sequence: GGAGAAAUUAUCCUUGGUGUGU - SEQ ID 15) or with mirVana™ miRNA mimic Negative Control #1, which has a unique sequence designed such that it does not target any human, mouse, or rat gene (Life technologies, Carlsbad, CA, USA) using DharmaFECT Duo transfection reagent (Thermo Fisher Scientific, Waltham, Massachusetts, USA) according to the manufacturer's instructions. Briefly, 1 µl of mirVana™ miRNA mimics (Stock solution 50 µM) and 4 µl of transfection reagent were diluted with 195 µL Opti-MEM (Invitrogen Carlsbad, CA, USA), and incubated for 20 min at room temperature, and then added to the cells. While lipid/DNA complexes were forming, the cell culture medium was removed and replaced with 800 µl OptiMEM. Transfection mix was added to the cells and after 4h the transfection cocktail was replaced with2ml normal cell culture medium or culture medium supplemented with 500 ng/ml doxycycline (Sigma, Munich, Germany) for 48 h to induce the production of HNF-1alpha. All Assays were done 24h or 48h after transfection.

### Quantitative real-time RT-PCR (RNA)

Total RNA was extracted using the miRNeasy mini Kit (Qiagen, Hilden, Germany). 1 µg total RNA was reverse-transcribed using 24 µg random hexamers (Thermo Fisher Scientific, Waltham, Massachusetts, USA), 0.5 mm dNTPs, 5× first-strand buffer, 0.01 mol/l dithiothreitol, and 200 units of SuperScript II reverse transcriptase (Invitrogen Carlsbad, CA, USA) in a final reaction volume of 20 µl. SYBR Green I-based real-time RT-PCR was performed on the TaqMan 7500 (Life technologies, Carlsbad, CA, USA) using the QuantiTech SYBR Green PCR kit (Qiagen, Hilden, Germany) as per manufacturers' protocol. The PCR was performed using the following thermal cycling protocol: 95°C for 15 min, 95°C for 15 sec following 58°C for 35s and 72°C for 45s for 40 cycles. In relative quantification the expression of Insulin mRNA was measured with respect to the reference gene β-actin, which was expressed constitutively and at the same level in all the samples analysed. The primers used to amplify insulin and β-actin (purchased from Sigma-Aldrich, St Louis, Missouri, USA) were: Insulin *for:* AACAGCACCTTTGTGGTCCT (SEQ ID 16) Insulin *rev:* GTGCAGCACTGATCCACAAT (SEQ ID 17); β-actin *for:* AGCCATCCAGGCTGTGTTGT (SEQ ID 18) and β-actin *rev:* CAGCTGTGGTGGTGAAGCTG (SEQ ID 19).

### Statistical analysis

Results of miRNA expression in cells were expressed as means ± SEM. Differences between treatments were analyzed by Student's t-test, as well as one-way analysis of variance (ANOVA) and subsequent Tukey's tests. Serum measurements and clinical data were given as median and inter quartile range (IQR) and compared by Mann-Whitney U-test and Spearman correlation analysis. Statistical analysis was conducted using SPSS (IBM Corp., Armonk, NY) and MatLab (The MathsWorks, Inc, Natick, MA). Differences were considered to be significant at *P*<0.05.

**Table 1**

| Signalling pathways of putative targets of miR-103, mir-503 and -224 were predicted using the gene functional classification tool DAVID. The p-value (a modified Fisher Exact P-Value which ranges from 0-1) highlights how strongly enriched in the annotation category the biological pathway is, where 0 represents perfect enrichment. This tool identified numerous pathways which involve a number of the predicted gene targets, and the top ten predicted gene pathways for each of the differentially expressed miRNAs are shown. | | | |
|---|---|---|---|
| **Pathways Involved** | **Count** | **%** | **P value** |
| | | | |

| **miR-103** | | | |
|---|---|---|---|
| Calcium signalling pathway | 18 | 3.1 | 1.0E⁻⁴ |
| Oocyte meiosis | 12 | 2.0 | 1.1E⁻³ |
| Leukocvte transendothelial migration | 12 | 2.0 | 1.4E⁻³ |
| Long-term potentiation | 9 | 1.5 | 1.6E⁻³ |
| GnRH signalling pathway | 10 | 1.7 | 3.8E⁻³ |
| Insulin signalling pathway | 12 | 2.0 | 4.3E⁻³ |
| Phosphatidylinositol signalling system | 8 | 1.4 | 8.2E⁻³ |
| Vascular smooth muscle contraction | 10 | 1.7 | 1.3E⁻² |
| Neuroactive ligand receptor interaction | 17 | 2.9 | 1.3E⁻² |
| | | | |

| **miR-224** | | | |
|---|---|---|---|
| Phosphatidylinositol signalling system | 8 | 1.9 | 1.8E⁻³ |
| TGF-β signalling pathway | 8 | 1.9 | 5.7E⁻³ |
| Fcgamma R mediated phagocytosis | 8 | 1.9 | 6.5E⁻³ |
| Long-term depression | 7 | 1.6 | 6.5E⁻³ |
| Long-term potentiation | 7 | 1.6 | 6.5E⁻³ |
| Oocvte meiosis | 9 | 2.1 | 6.7E⁻³ |
| Endocytosis | 12 | 2.8 | 1.2E⁻² |
| Axon guidance | 9 | 2.1 | 1.4E⁻² |
| Melangoenesis | 7 | 1.6 | 2.8E⁻² |
| GnRH signalling pathway | 7 | 1.6 | 3.1E⁻² |
| | | | |
| | | | |
| | | | |

| **miR-503** | | | |
|---|---|---|---|
| Pancreatic cancer | 8 | 2.2 | 5.7E⁻⁴ |
| Insulin signalling pathway | 10 | 2.8 | 1.8E⁻³ |
| Glycerophospholipid metaboilsm | 7 | 1.9 | 1.8E⁻³ |
| TGF-β signalling pathway | 8 | 2.2 | 2.1E⁻³ |
| Fcgamma R mediated phagocytosis | 8 | 2.2 | 2.4E⁻³ |
| Long-term depression | 7 | 1.9 | 2.7E⁻³ |
| Prostate cancer | 8 | 2.2 | 2.7E⁻³ |
| Melanoma | 7 | 1.9 | 3.2E⁻³ |
| Chronic myeloid leukemia | 7 | 1.9 | 4.8E⁻³ |
| Pathways in cancer | 15 | 4.2 | 6.5E⁻³ |

**Table 2: Clinical data of HNF1A-MODY, HNF1A-MODY negative family members and T2DM patients.**

| | **HNF1A-MODY** | **HNF1A-MODY negative family** | **T2DM** | **Statistical tests** | | |
|---|---|---|---|---|---|---|
| **N** | 31 | 10 | 17 | | | |

| | **Median (inter quartile range)** | | | **Kruskal Wallis p-value** | | **Post hoc significance** |
|---|---|---|---|---|---|---|
| **Age [yrs]** | 36 (21-52) | 24 (18-46) | 52 (43-66) | 0.0030 | * | T2DM vs others |
| **Duration [yrs]** | 6 (2-19) | N/A | N/A | N/A | | |
| **BMI [kg/m²]** | 24.4 (22.1-26.2) | 22.8 (20.4-30.5) | 26.8 (25.3-27.4) | 0.1062 | n.s. | |
| **HbA_{1c} [%]** | 7.1 (6.3-8.0) | 5.3 (5.2-5.5) | 6.9 (6.6-7.0) | 0.00002 | * | HNF1A-negative vs others |
| **SBP [mmHg]** | 120.5 (114.0-130.0) | 112.5 (111.0-140.0) | 130 (127.5-143.8) | 0.0978 | n.s. | |
| **DBP [mmHg]** | 70.5 (67.0-80.0) | 69 .0 (64.0-85.0) | 80 (75.3-85.0) | 0.0727 | n.s. | |
| **TG [mmol/L]** | 0.71 (0.60-0.90) | 0.90 (0.70-1.11) | 1.19 (0.81-2.12) | 0.0310 | * | HNF1A vs T2DM |
| **Chol [mmol/L]** | 4.3 (3.6-5.0) | 4.6 (4.1-5.1) | 4.1 (3.5-4.5) | 0.4338 | n.s. | |
| **HDL [mmol/L]** | 1.4 (1.1-1.7) | 1.11 (0.8-1.4) | 1.1 (0.9-1.3) | 0.1429 | n.s. | |
| **LDL [mmol/L]** | 2.5 (2.1-3.1) | 3.2 (2.6-3.6) | 1.9 (1.8-2.5) | 0.0362 | * | T2DM vs others |
| **OGIS [ml min⁻¹m⁻²]** | 349.0 (289.3-427.5) | 501.5 (414.0-512.0) | 318.0 (287.5-351.5) | 0.0020 | * | HNF1A-negative vs others |
| **Fasting glucose [mmol/L]** | 6.8 (5.3-8.5) | 4.8 (4.6-5.0) | 7.0 (6.3-7.4) | 0.0001 | * | HNF1A-negative vs others |
| **AUC glucose [mmol/L]** | 56.15 (44.19-68.99) | 25.90 (24.25-27.00) | 43.40 (29.23-53.49) | 0.000003 | * | between all groups |
| **AUC insulin [pmil/L]** | 43.28 (27.86-73.80) | 180.17 (118.25-233.90) | 77.13 (56.33-115.03) | 0.0006 | * | HNF1A vs others |
| **miR_103** | 1030 (457-3317) | 14 (0-126) | 129 (8-399) | 0.0000003 | * | HNF1A vs others |
| **miR_224** | 2610 (9-18475) | 33 (22-46) | N/A | 0.0451 | * | HNF1A vs HNF1A-negative |

**Table 3: Correlations of miR-103 and miR-224 with clinical parameters in HNF1A-MODY patients**

| **miR-103 Clinical Parameter** | **Correlation** | **P value** |
|---|---|---|
| Age (yrs) | rho= -0.21 | 0.25 |
| Duration of diabetes (yrs) | rho= -0.02 | 0.92 |
| HbA_{1c} (%) | rho= 0.12 | 0.54 |
| Systolic Blood pressure(mmHg) | rho= -0.45 | **0.01** |
| Triglycerides (mmol/L) | rho= -0.44 | **0.02** |
| OGIS (ml min⁻¹m⁻²) | rho= 0.18 | 0.34 |

| **miR-224 Clinical Parameter** | **Correlation** | **P value** |
|---|---|---|
| Age (yrs) | rho= 0.09 | 0.64 |
| Duration of diabetes (yrs) | rho= -0.17 | 0.35 |
| HbA_{1c} (%) | rho= -0.16 | 0.39 |
| Systolic Blood pressure(mmHg) | rho= -0.02 | 0.93 |
| Triglycerides (mmol/L) | rho= -0.09 | 0.64 |
| | rho= 0.44 | **0.01** |

**Table 4**

| |
|---|
| hsa-miR-103a-3p mature sequence: |
| AGCAGCAUUGUACAGGGCUAUGA (SEQ ID NO: 1) |
| |
| hsa-miR-103a-1 precursor sequence: |
| UACUGCCCUCGGCUUCUUUACAGUGCUGCCUUGUUGCAUAUGGAUCAA GCAGCAUUGUACAGGGCUAUGAAGGCAUUG (SEQ ID NO: 7) |
| |
| hsa-miR-224-5p mature sequence: |
| CAAGUCACUAGUGGUUCCGUU (SEQ ID NO: 3) |
| |
| hsa-miR-224 precursor sequence: |
| GGGCUUUCAAGUCACUAGUGGUUCCGUUUAGUAGAUGAUUGUGCAUUG UUUCAAAAUGGUGCCCUAGUGACUACAAAGCCC (SEQ ID NO: 8) |
| |
| hsa-miR-551b mature sequence: |
| GCGACCCAUACUUGGUUUCAG (SEQ ID NO: 2) |
| |
| hsa-miR-551b precursor sequence: |
| AGAUGUGCUCUCCUGGCCCAUGAAAUCAAGCGUGGGUGAGACCUGGUG CAGAACGGGAAGGCGACCCAUACUUGGUUUCAGAGGCUGUGAGAAUAA (SEQ ID NO: 9) |
| |
| hsa-miR-503-5p mature sequence: |
| UAGCAGCGGGAACAGUUCUGCAG (SEQ ID NO: 4) |
| |
| hsa-miR-503 precursor: |
| UGCCCUAGCAGCGGGAACAGUUCUGCAGUGAGCGAUCGGUGCUCUGGG GUAUUGUUUCCGCUGCCAGGGUA (SEQ ID NO: 10) |
| |
| hsa-miR-539 mature sequences: |
| UACUGCAGACAGUGGCAAUCA (has-miR-539-5p: SEQ ID NO: 12) or |
| UGAUUGGUACGUCUGUGGGUAG (has-miR-539-3p: SEQ ID NO: 5) |
| |
| hsa-miR-509 precursor: |
| CAUGCUGUGUGUGGUACCCUACUGCAGACAGUGGCAAUCAUGUAUAAU UAAAAAUGAUUGGUACGUCUGUGGGUAGAGUACUGCAUGACACAUG (SEQ ID NO: 11) |

**Table 5**

| Clinical data of HNF4A-MODY patients: | | |
|---|---|---|
| **Clinical characteristics of HNF4A-MODY subjects** | | |
| | **HNF4A-MODY median (IQR)** | **P value compared to HNF1A-MODY** |
| **n (male/female)** | 9 (4/5) | 0.91 NS |
| **Age (years)** | 32 (23-46) | 0.57 NS |
| **Duration of diabetes (years)** | 7 (0-16) | 0.42 NS |
| **Body mass index (kg/m²)** | 23.9 (21.8-25.0) | 0.55 NS |
| **Systolic blood pressure (mmHg)** | 115 (109-129) | 0.27 NS |
| **Diastolic blood pressure (mmHg)** | 73 (67-79) | 0.83 NS |
| **Total cholesterol (mmol/L)** | 3.7 (3.4-4.3) | 0.12 NS |
| **LDL cholesterol (mmol/L)** | 2.2 (1.8-2.7) | 0.14 NS |
| **HDL cholesterol (mmol/L)** | 1.2 (0.8-1.6) | 0.28 NS |
| **TG (mmol/L)** | 0.62 (0.52-0.79) | 0.23 NS |
| **HbA_{1c} (%)** | 5.7 (5.3-7.1) | 0.045 * |
| **Fasting plasma glucose (mmol/L)** | 5.0 (3.8-7.2) | 0.09 NS |
| **WCC (x10⁹/L)** | 6.66 (5.94-7.89) | 0.71 NS |
| **Neutrophil count (x10⁹/L)** | 3.70 (3.07-4.47) | 0.21 NS |
| **Patients treated with aspirin (n)** | 2 | 0.63 NS |
| **Patients treated with statin (n)** | 1 | 0.40 NS |

| | | |
|---|---|---|
| Results are expressed in median and interquartile range (IQR). *: significant; NS: not significant; LDL/HDL: low-density/high-density lipoprotein; TG: triglycerides; WCC: white cell count; Mann-Whitney *U* test for HNF1A-MODY vs. HNF4A-MODY revealed no statistically significant differences but for HbA_{1c}. Male/female ratio and treatment with aspirin or statins were tested for statistical difference using the χ² test. | | |

### References

[1] Shields BM, Hicks S, Shepherd MH, Colclough K, Hattersley AT, Ellard S (2010): Maturity-onset diabetes of the young (MODY): how many cases are we missing? Diabetologia 53:2504-2508.
[2] Wang H, Antinozzi PA, Hagenfeldt KA, Maechler P, Wollheim CB (2000) Molecular targets of a human HNF1 alpha mutation responsible for pancreatic beta-cell dysfunction. EMBO J 19: 4257-4264
[3] Yamagata K, Furuta H, Oda N, et al. (1996) Mutations in the hepatocyte nuclear factor-4alpha gene in maturity-onset diabetes of the young (MODY1). Nature 384: 458-460
[4] Bonner C, Farrelly AM, Concannon CG, et al. (2011) Bone morphogenetic protein 3 controls insulin gene expression and is down-regulated in INS-1 cells inducibly expressing a hepatocyte nuclear factor 1A-maturity-onset diabetes of the young mutation. J Biol Chem 286: 25719-25728
[5] Wang H, Maechler P, Hagenfeldt KA, Wollheim CB (1998) Dominant-negative suppression of HNF-1alpha function results in defective insulin gene transcription and impaired metabolism-secretion coupling in a pancreatic beta-cell line. EMBO J 17: 6701-6713
[6] Wobser H, Dussmann H, Kogel D, et al. (2002) Dominant-negative suppression of HNF-1 alpha results in mitochondrial dysfunction, INS-1 cell apoptosis, and increased sensitivity to ceramide-, but not to high glucose-induced cell death. J Biol Chem 277: 6413-6421
[7] Asfari M, Janjic D, Meda P, Li G, Halban PA, Wollheim CB (1992) Establishment of 2-mercaptoethanol-dependent differentiated insulin-secreting cell lines. Endocrinology 130: 167-178
[8] Huang da W, Sherman BT, Lempicki RA (2009) Systematic and integrative analysis of large gene lists using DAVID bioinformatics resources. Nat Protoc 4: 44-57
[9] Dennis G, Jr., Sherman BT, Hosack DA, et al. (2003) DAVID: Database for Annotation, Visualization, and Integrated Discovery. Genome Biol 4: P3
[10] Kyithar MP, Bacon S, Pannu KK, et al. (2011) Identification of HNF1A-MODY and HNF4A-MODY in Irish families: phenotypic characteristics and therapeutic implications. Diabetes Metab 37: 512-519
[11] Ellard S, Colclough K (2006) Mutations in the genes encoding the transcription factors hepatocyte nuclear factor 1 alpha (HNF1A) and 4 alpha (HNF4A) in maturity-onset diabetes of the young. Hum Mutat 27: 854-869
[12] Mari A, Pacini G, Murphy E, Ludvik B, Nolan JJ (2001) A model-based method for assessing insulin sensitivity from the oral glucose tolerance test. Diabetes Care 24: 539-548

## Claims

1. A method for determining whether an individual is a HNF1A-MODY carrier or a diabetes patient with a defect in HNF1A signalling, comprising a step of assaying a biological sample from the individual to:
(a) detect increased abundance of a micro RNA molecule selected from miR103, miR551b, or miR224; and/or
(b) detect decreased abundance of a micro RNA molecule selected from miR503, and miR539,
wherein increased abundance of one or more of miR103, miR551b, or miR224 and/or decreased abundance of one or more of miR503 and miR539, indicates that the individual is a HNF1A-MODY carrier or in which increased abundance of one or more of miR103, miR551b or miR224 indicates that the individual is a diabetes patient with a defect in HNF1A signalling.

2. A method for distinguishing between a HNF1A-MODY carrier and a body mass index (BMI)-matched Type-2 diabetes patient, the method comprising a step of assaying a biological sample from the individual to detect an abundance of miR103 or miR224, wherein the abundance of miR-103 or miR224 is lower in a type-2 diabetes patient compared to a HNF1A-MODY carrier.

3. An (a) inhibitor of miR551b, and/or (b) miR503 or miR539 in a primary, mature or precursor form, or a mimic thereof, for use in a method of treatment of a metabolic disorder in a patient, wherein the metabolic disorder is **characterised by** a deficiency in insulin secretion selected from HNF1A-MODY, HNF4A-MODY and Type-2 Diabetes

4. An (a) inhibitor of miR551b, and/or (b) miR503 or miR539 in a primary, mature or precursor form, or a mimic thereof, for use of Claim 3 for use in:: decreasing, or delaying onset of increase in, blood sugar levels; decreasing gluconeogenesis, improving insulin sensitivity, preventing or delaying onset of insulin resistance, improving glucose tolerance, increasing insulin levels.

5. An (a) inhibitor of miR551b, and/or (b) miR503 or miR539 in a primary, mature or precursor form, or a mimic thereof, for use of Claim 3 or 4, wherein said method comprises an initial step of identifying a patient suitable for treatment comprising the steps of:
(a)detecting increased abundance of a micro RNA molecule selected from miR103, miR551b, or miR224; or
(b)detecting decreased abundance of a micro RNA molecule selected from miR503, and miR539,
wherein detection of increased abundance of one or more of miR103, miR551b, or miR224, or detection of decreased abundance of one or more of miR503 and miR539, indicates that the individual is suitable for treatment.

6. An (a) inhibitor of miR103, miR551b or (b) miR503 or miR539 in a primary, mature or precursor form, or a mimic thereof, as a medicament.

7. A pharmaceutical composition comprising an active agent and a pharmaceutically acceptable carrier, in which the active agent is selected from (a) an inhibitor of miR103, miR551b, and/or (b) miR503 and/or miR539 in a primary, mature or precursor form, or a mimic thereof.

8. A pharmaceutical composition as claimed in Claim 7 in which the active agent is an inhibitor of miR103.

9. A method for determining a suitable treatment for an individual with HNF1A-MODY, the method comprising a step of detecting increased abundance of a micro RNA molecule selected from miR224, miR551b or miR103, wherein detection of increased abundance of one or more of miR224, miR551b or miR103 indicates that the individual is suitable for treatment with a therapy that stimulates insulin production or insulin secretion.

10. The method of Claim 9, in which the suitable treatment is sulfonylureas.

## Patentansprüche

1. Verfahren für die Bestimmung, ob ein Individuum ein HNF1A-MODY-Träger oder ein Diabetespatient mit einem Defekt in der HNF1A-Signalgebung ist, das einen Schritt der Untersuchung einer biologischen Probe von dem Individuum umfasst, um:
(a) eine erhöhte Abundanz eines mikro-RNA-Moleküls nachzuweisen, das aus miR103, miR551b oder miR224 ausgewählt ist; und/oder
(b) eine verringerte Abundanz eines mikro-RNA-Moleküls nachzuweisen, das aus miR503 und miR539 ausgewählt ist,
wobei eine erhöhte Abundanz von einem oder mehreren von miR103, miR551b oder miR224 und/oder verringerte Abundanz von einem oder mehreren von miR503 und miR539 anzeigt, dass das Individuum ein HNF1A-MODY-Träger ist, oder in welchem eine erhöhte Abundanz von einem oder mehreren von miR103, miR551b oder miR224 anzeigt, dass das Individuum ein Diabetespatient mit einem Defekt in der HNF1A-Signalgebung ist.

2. Verfahren für die Unterscheidung zwischen einem HNF1A-MODY-Träger und einem Typ-2-Diabetespatient mit passendem Body-Mass-Index (BMI), wobei das Verfahren einen Schritt der Untersuchung einer biologischen Probe von dem Individuum umfasst, um eine Abundanz von miR103 oder miR224 nachzuweisen, wobei die Abundanz von miR103 oder miR224 bei einem Typ-2-Diabetespatienten im Vergleich zu einem HNF1A-MODY-Träger geringer ist.

3. (a) Inhibitor von miR551b und/oder (b) miR503 oder miR539 in einer primären, reifen oder Vorläuferform oder einem Imitator davon für die Verwendung in einem Verfahren zur Behandlung einer Stoffwechselstörung bei einem Patienten, wobei die Stoffwechselstörung durch einen Mangel an Insulinsekretion gekennzeichnet ist, die aus HNF1A-MODY, HNF4A-MODY und Typ-2-Diabetes ausgewählt ist.

4. (a) Inhibitor von miR551b und/oder (b) miR503 oder miR539 in einer primären, reifen oder Vorläuferform oder einem Imitator davon für die Verwendung nach Anspruch 3 für die Verwendung bei: Verringerung oder Verzögerung des Beginns der Zunahme der Blutzuckerspiegel, Verringerung von Gluconeogenese, Verbesserung von Insulinsensitivität, Prävention oder Verzögerung des Beginns von Insulinresistenz, Verbesserung von Glucosetoleranz, Erhöhung von Insulinspiegeln.

5. (a) Inhibitor von miR55 1b und/oder (b) miR503 oder miR539 in einer primären, reifen oder Vorläuferform oder einem Imitator davon für die Verwendung nach Anspruch 3 oder 4, wobei das Verfahren einen Anfangsschritt der Identifizierung eines Patienten, der für die Behandlung geeignet ist, umfasst, der die folgenden Schritte umfasst:
(a) Nachweisen von erhöhter Abundanz eines mikro-RNA-Moleküls, das aus miR103, miR551b oder miR224 ausgewählt ist; oder
(b) Nachweisen von verringerter Abundanz eines mikro-RNA-Moleküls, das aus miR503 und miR539 ausgewählt ist,
wobei der Nachweis von erhöhter Abundanz von einem oder mehreren von miR103, miR551b oder miR224 oder der Nachweis von verringerter Abundanz von einem oder mehreren von miR503 und miR539 anzeigt, dass das Individuum für die Behandlung geeignet ist.

6. (a) Inhibitor von miR103, miR551b oder (b) miR503 oder miR539 in einer primären, reifen oder Vorläuferform oder einem Imitator davon als Medikament.

7. Pharmazeutische Zusammensetzung, die einen Wirkstoff und einen pharmazeutisch verträglichen Träger umfasst, in welcher der Wirkstoff ausgewählt ist aus: (a) einem Inhibitor von miR103, miR551b und/oder (b) miR503 und/oder miR539 in einer primären, reifen oder Vorläuferform oder einem Imitator davon.

8. Pharmazeutische Zusammensetzung nach Anspruch 7, in welcher der Wirkstoff ein Inhibitor von miR103 ist.

9. Verfahren für die Bestimmung einer geeigneten Behandlung für ein Individuum mit HNF1A-MODY, wobei das Verfahren einen Schritt des Nachweises von erhöhter Abundanz eines mikro-RNA-Moleküls umfasst, das aus miR224, miR551b oder miR103 ausgewählt ist, wobei der Nachweis von erhöhter Abundanz von einem oder mehreren von miR224, miR551b oder miR103 anzeigt, dass das Individuum für die Behandlung mit einer Therapie, die die Insulinproduktion oder Insulinsekretion stimuliert, geeignet ist.

10. Verfahren nach Anspruch 9, in welchem die geeignete Behandlung Sulfonylharnstoffe ist.

## Revendications

1. Méthode de détermination si un individu est ou non un porteur de HNF1A-MODY ou un patient diabétique ayant un défaut de signalisation de HNF1A, comprenant une étape de dosage d'un échantillon biologique issu de l'individu afin de :
(a) détecter l'abondance accrue d'une molécule de microARN choisie parmi miR103, miR551b, ou miR224 ; et/ou
(b) détecter l'abondance réduite d'une molécule de microARN choisie parmi miR503, et miR539,
dans laquelle l'abondance accrue d'un ou plusieurs parmi miR103, miR551b, ou miR224, et/ou l'abondance réduite d'un ou plusieurs parmi miR503 et miR539, indique que l'individu est un porteur de HNF1A-MODY, ou où l'abondance accrue d'un ou plusieurs parmi miR103, miR551b, ou miR224 indique que l'individu est un patient diabétique ayant un défaut de signalisation HNF1A.

2. Méthode de distinction entre un porteur de HNF1A-MODY et un patient souffrant d'un diabète de type 2 jumelé à un indice de masse corporelle (IMC), la méthode comprenant une étape de dosage d'un échantillon biologique issu de l'individu afin de détecter une abondance de miR103 ou miR224, où l'abondance de miR103 ou miR224 est inférieure chez un patient souffrant d'un diabète de type 2 par rapport à un porteur de HNF1A-MODY.

3. (a) Inhibiteur de miR551b, et/ou (b) miR503 ou miR539 sous forme primaire, mature ou de précurseur, ou un mimétique de celui-ci, pour une utilisation dans une méthode de traitement d'un trouble métabolique chez un patient, où le trouble métabolique est **caractérisé par** une déficience de sécrétion d'insuline choisie parmi HNF1A-MODY, HNF4A-MODY et le diabète de type 2.

4. (a) Inhibiteur de miR551b, et/ou (b) miR503 ou miR539 sous forme primaire, mature ou de précurseur, ou un mimétique de celui-ci, pour une utilisation selon la revendication 3, destiné à une utilisation dans : la diminution, ou le retardement du déclenchement d'une augmentation des taux de glycémie ; la diminution de la gluconéogenèse, l'amélioration de la sensibilité à l'insuline, la prévention ou le retardement du déclenchement d'une insulinorésistance, l'amélioration de la tolérance au glucose, l'augmentation des taux d'insuline.

5. (a) Inhibiteur de miR551b, et/ou (b) miR503 ou miR539 sous forme primaire, mature ou de précurseur, ou un mimétique de celui-ci, pour une utilisation selon la revendication 3 ou 4, où ladite méthode comprend une étape initiale d'identification d'un patient convenable pour le traitement, comprenant les étapes consistant à :
(a) détecter l'abondance accrue d'une molécule de microARN choisie parmi miR103, miR551b, ou miR224 ; ou
(b) détecter l'abondance réduite d'une molécule de microARN choisie parmi miR503, et miR539,
où la détection de l'abondance accrue d'un ou plusieurs parmi miR103, miR551b, ou miR224, ou la détection de l'abondance réduite d'un ou plusieurs parmi miR503 et miR539, indique que l'individu est convenable pour le traitement.

6. (a) Inhibiteur de miR103, miR551b, ou (b) miR503 ou miR539 sous forme primaire, mature ou de précurseur, ou un mimétique de celui-ci, comme médicament.

7. Composition pharmaceutique comprenant un agent actif et un véhicule pharmaceutiquement acceptable, dans laquelle l'agent actif est choisi parmi (a) un inhibiteur de miR103, miR551b, et/ou (b) miR503 et/ou miR539 sous forme primaire, mature ou de précurseur, ou un mimétique de celui-ci.

8. Composition pharmaceutique selon la revendication 7, dans laquelle l'agent actif est un inhibiteur de miR103.

9. Méthode de détermination d'un traitement convenable pour un individu porteur de HNF1A-MODY, la méthode comprenant une étape consistant à détecter l'abondance accrue d'une molécule de microARN choisie parmi miR224, miR551b ou miR103, où la détection de l'abondance accrue d'un ou plusieurs parmi miR224, miR551b ou miR103 indique que l'individu est convenable pour un traitement par une thérapie qui stimule la production d'insuline ou la sécrétion d'insuline.

10. Méthode selon la revendication 9, dans laquelle le traitement convenable est à base de sulfonylurées.
